(19) Europäisches Patentamt / European Patent Office / Office européen des brevets

(11) **EP 4 357 369 B1**

(12) **EUROPEAN PATENT SPECIFICATION**

(45) Date of publication and mention
of the grant of the patent:
**17.06.2026 Bulletin 2026/25**

(21) Application number: **22824241.8**

(22) Date of filing: **15.06.2022**

(51) International Patent Classification (IPC):
*A61K 40/11* (2025.01)     *A61K 40/30* (2025.01)
*A61K 40/31* (2025.01)     *A61K 40/42* (2025.01)
*C07K 19/00* (2006.01)     *C12N 5/10* (2006.01)
*C12N 15/867* (2006.01)    *A61K 39/00* (2006.01)
*A61P 35/00* (2006.01)     *A61P 35/04* (2006.01)
*C07K 14/725* (2006.01)    *C12N 5/0783* (2010.01)
*C12N 9/64* (2006.01)      *C12N 15/86* (2006.01)
*A61P 35/02* (2006.01)

(52) Cooperative Patent Classification (CPC):
**A61K 40/11; A61K 40/31; A61K 40/4211;
A61K 40/4262; A61P 35/02; A61P 35/04;
C07K 14/7051; C12N 5/0636; C12N 5/0646;
C12N 5/10; C12N 9/6467; C12Y 304/21079;**
A61K 2239/15; A61K 2239/28; A61K 2239/31;
                                          (Cont.)

(86) International application number:
**PCT/CN2022/098911**

(87) International publication number:
**WO 2022/262765 (22.12.2022 Gazette 2022/51)**

(54) **PREPARATION AND APPLICATION OF CHIMERIC ANTIGEN RECEPTOR IMMUNE CELL CONSTRUCTED ON BASIS OF GRANZYME B**

HERSTELLUNG UND ANWENDUNG EINER CHIMÄREN ANTIGENREZEPTOR-IMMUNZELLE AUF BASIS VON GRANZYM B

PRÉPARATION ET UTILISATION D'UNE CELLULE IMMUNITAIRE DE RÉCEPTEUR ANTIGÉNIQUE CHIMÉRIQUE CONSTRUITE SUR LA BASE DE GRANZYME B

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.06.2021 CN 202110667439**

(43) Date of publication of application:
**24.04.2024 Bulletin 2024/17**

(73) Proprietor: **West China Hospital, Sichuan University
Chengdu, Sichuan 610041 (CN)**

(72) Inventors:
• **ZHAO, Xudong**
  **Chengdu, Sichuan 610041 (CN)**
• **SUN, Bin**
  **Chengdu, Sichuan 610041 (CN)**

(74) Representative: **Santoro, Sofia et al
Società Italiana Brevetti S.p.A.
Via Giosuè Carducci, 8
20123 Milano (IT)**

(56) References cited:
WO-A1-2016/120217    WO-A1-2016/120217
WO-A1-2019/043170    WO-A2-2004/018002
CN-A- 1 555 268

- GEHRMANN MATHIAS ET AL: "Immunotherapeutic Targeting of Membrane Hsp70-Expressing Tumors Using Recombinant Human Granzyme B", vol. 7, no. 7, 19 July 2012 (2012-07-19), pages e41341, XP093015802, Retrieved from the Internet <URL:https://journals.plos.org/plosone/article/file?id=10.1371/journal.pone.0041341&type=printable> DOI: 10.1371/journal.pone.0041341
- GROSS CATHARINA ET AL: "Cell Surface-bound Heat Shock Protein 70 (Hsp70) Mediates Perforin-independent Apoptosis by Specific Binding and Uptake of Granzyme B", JOURNAL OF BIOLOGICAL CHEMISTRY, vol. 278, no. 42, 1 October 2003 (2003-10-01), US, pages 41173 - 41181, XP093238643, ISSN: 0021-9258, DOI: 10.1074/jbc.M302644200
- GEHRMANN MATHIAS, STANGL STEFAN, KIRSCHNER ANDREAS, FOULDS GEMMA A., SIEVERT WOLFGANG, DOSS BRIGITTE T., WALCH AXEL, POCKLEY ALAN : "Immunotherapeutic Targeting of Membrane Hsp70-Expressing Tumors Using Recombinant Human Granzyme B", PLOS ONE, vol. 7, no. 7, 19 July 2012 (2012-07-19), pages e41341, XP093015802, DOI: 10.1371/journal.pone.0041341
- BIOLOGICAL ABSTRACTS, 1 January 1900, Philadelphia, PA, US; abstract no. PREV200300583456, GROSS C, ET AL: "Cell surface-bound heat shock protein 70 (Hsp70) mediates perforin-independent apoptosis by specific binding and uptake of granzyme B." XP002276406
- STANGL, S. ET AL.: "Targeting Membrane Heat-shock Protein 70 (Hsp70) on Tumors by cmHsp70.1 Antibody", PNAS, vol. 108, no. 2, 11 January 2011 (2011-01-11), XP055261079, DOI: 10.1073/pnas.1016065108
- PRANAV OBEROI, ROBERT JABULOWSKY, WINFRIED WELS: "Selective Induction of Cancer Cell Death by Targeted Granzyme B", ANTIBODIES, vol. 2, no. 4, pages 130 - 151, XP055714373, DOI: 10.3390/antib2010130
- SHEVTSOV MAXIM, HUILE GAO, MULTHOFF GABRIELE: "Membrane heat shock protein 70: a theranostic target for cancer therapy", PHILOSOPHICAL TRANSACTIONS. ROYAL SOCIETY OF LONDON. B: BIOLOGICAL SCIENCES., ROYAL SOCIETY, LONDON., GB, vol. 373, no. 1738, 19 January 2018 (2018-01-19), GB
, pages 20160526, XP093015803, ISSN: 0962-8436, DOI: 10.1098/rstb.2016.0526
- DATABASE GenBank 18 September 1995 (1995-09-18), HADDAD, P. ET AL.: "Granzyme B [Homo Sapiens] ", XP009541989, Database accession no. AAA7549.1
- ZHANG PING, ZHAI JIANZHAO;LIU ZAISHUAN;ZHAO XUDONG;WU YONGKANG: "Advances in Chimeric Antigen Receptor T Cell Immunotherapy Construction Technology", INTERNATIONAL JOURNAL OF LABORATORY MEDICINE, CN, vol. 42, no. 9, 31 May 2021 (2021-05-31), CN
, pages 1125 - 1129, XP093015806, ISSN: 1673-4130, DOI: 10.3969/j.issn.1673-4130.2021.09.024

Remarks:
    The complete document including Reference Table(s) and the Sequence Listing(s) can be downloaded from the EPO website

(52)  Cooperative Patent Classification (CPC): (Cont.)
    A61K 2239/38; A61K 2239/54; A61K 2239/55;
    C07K 2319/03; C12N 15/86; C12N 2740/16043

**Description**

**Technical field**

The present invention belongs to the field of tumor immunotherapy biomedicine technology, and relates to specific chimeric antigen receptor immune cells. Specifically, it relates to a CAR constructed based on GrB, an immune response cell modified with the same, and the preparation method and application thereof.

**Background**

Tumor is the second major disease that threatens human health. In 2018, there were 18,100,000 new cancer patients and 9,500,000 tumor deaths worldwide. It is estimated that by 2040, there will be 29,500,000 new cases and 16,400,000 deaths of cancer per year. Although conventional tumor treatment methods such as radiotherapy, chemotherapy, and surgical resection are able to prolong the survival of cancer patients, they are still constrained by the decline in life quality of patients and high risk of recurrence.

At the 2012 Annual Conference of the International Society for Cellular Therapy, it was pointed out that bioimmunotherapy has become the fourth cancer treatment method after surgery, radiotherapy and chemotherapy, and will become a must-have method for future cancer treatment. Chimeric Antigen Receptor-T (CAR-T) cells refer to T cells that are genetically modified to be able to recognize specific target antigens in a non MHC-restricted manner, with continuous activation and amplification. The structure of CAR comprises a tumor associated antigen binding region, an extracellular hinge region, a transmembrane region and an intracellular signaling region. At present, CART therapy has shown strong killing ability in hematological malignancies, but the application of CART therapy in solid tumors is limited due to tumor heterogeneity, lack of tumor specific antigens, and tumor immunosuppressive microenvironment.

Heat shock response is a self-protection mechanism produced by cells or tissues in the face of various stress stimuli, often accompanied by upregulation of heat shock proteins (HSPs) expression. HSP70, as the most important heat shock protein in humans, is low expressed and localized within cells under normal physiological conditions, but overexpressed in tumor cells to promote cell proliferation, inhibit cell aging, and resist stress-induced cell apoptosis. Inhibiting HSP70 in tumor cells can lead to cell death and the formation of transplanted tumors in nude mouse. HSP70 is highly expressed in most tumor samples, which can be used as a molecular marker of early prostate cancer and liver cancer, and is related to the prognosis of various tumors such as acute myeloid leukemia, breast cancer, endometrial cancer and rectal cancer. Methods targeting HSP70 expression, such as shRNA, CRISPR/Cas9 knockout, aptamers, inhibitors and the like are expected to be new approaches for cancer treatment. However, due to the complex heat shock protein family in eukaryotes, and the certain functional compensation among members of the heat shock protein family, other members of the HSPs family may compensate for the effect of HSP70 while the above methods reduce the expression of HSP70. WO2016120217A1 describes CARs targeting HSP70 comprising scFvs derived from anti-HSP70 specific antibodies, showing that these CARs specifically target membrane expressed HSP70, and are mentioned to be useful for immunotherapy of acute myeloid leukemia, colorectal cancer, lung cancer, neuronal cancer, pancreatic carcinomas, liver metastases or head-and-neck cancer.

Therefore, there is an urgent need to develop a chimeric antigen receptor immune cell targeting heat shock protein and a therapeutic method using the same in this field.

**Summary of the invention**

The purpose of the invention is to provide a chimeric antigen receptor immune cell constructed based on Granzyme B, the preparation thereof and the therapy using the same.

In the first aspect of the present invention, it provides a chimeric antigen receptor (CAR) modified based on Granzyme B (GrB), wherein the CAR comprises an extracellular binding domain that specifically binds to a heat shock protein. wherein the extracellular binding domain comprises a GrB protein or a fragment thereof which has an amino acid sequence as shown in SEQ ID NO: 1, or an amino acid sequence as shown in positions 21-247 of SEQ ID NO: 1.

In another preferred embodiment, the heat shock protein is selected from a group consisting of: HSP70, HSP60, HSP90, and a combination thereof.

In another preferred embodiment, the heat shock protein is a heat shock protein located on the cell membrane (or membrane-bound) (mHSP).

In another preferred embodiment, the heat shock protein is selected from a group consisting of: mHSP70, mHSP60, mHSP90, and a combination thereof.

In another preferred embodiment, the heat shock protein is HSP70, preferably mHSP70.

In another preferred embodiment, the heat shock protein is of human origin.

In another preferred embodiment, the amino acid sequence of the GrB protein or the fragment thereof is as shown in positions 21-247 of the sequence of SEQ ID NO: 1.

In another preferred embodiment, the structure of the CAR is shown in the following Formula I:

L-EB-H-TM-C-CD3ζ-RP (I)

wherein,

each "-" is independently a linking peptide or peptide bond;
L is absent or a signal peptide sequence;
EB is an extracellular binding domain;
H is absent or a hinge region;
TM is a transmembrane domain;
C is absent or a co-stimulatory signaling molecule;
CD3ζ is a cytoplasmic signal transduction sequence derived from CD3ζ;
RP is absent or a reporter protein.

In another preferred embodiment, the reporter protein RP further comprises a self splicing recognition site located at the N-terminus, preferably a T2A sequence.

In another preferred embodiment, the reporter protein RP is a fluorescent protein.

In another preferred embodiment, the reporter protein RP is a mKate2 red fluorescent protein.

In another preferred embodiment, the amino acid sequence of the mKate2 red fluorescent protein is shown in SEQ ID NO: 2.

In another preferred embodiment, L is a signal peptide of a protein selected from a group consisting of CD8, CD28, GM-CSF, CD4, CD137 and a combination thereof.

In another preferred embodiment, L is a signal peptide derived from CD8.

In another preferred embodiment, the amino acid sequence of L is shown in SEQ ID NO: 3.

In another preferred embodiment, H is a hinge region of a protein selected from a group consisting of CD8, CD28, CD137 and a combination thereof.

In another preferred embodiment, H is a hinge region derived from CD8.

In another preferred embodiment, the amino acid sequence of H is shown in SEQ ID NO: 4.

In another preferred embodiment, TM is a transmembrane region of a protein selected from a group consisting of CD28, CD3 epsilon, CD45, CD4, CD5, CD8, CD9, CD16, CD22, CD33, CD37, CD64, CD80, CD86, CD134, CD137, CD154 and a combination thereof.

In another preferred embodiment, TM is a transmembrane region derived from CD28.

In another preferred embodiment, the amino acid sequence of TM is shown in SEQ ID NO: 5.

In another preferred embodiment, C is a co-stimulatory signaling molecule of a protein selected from a group consisting of OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2 and a combination thereof.

In another preferred embodiment, C is a co-stimulatory signaling molecule derived from 4-1BB.

In another preferred embodiment, the amino acid sequence of C is shown in SEQ ID NO: 6.

In another preferred embodiment, the amino acid sequence of the cytoplasmic signal transduction sequence derived from CD3ζ is shown in SEQ ID NO: 7.

In another preferred embodiment, the amino acid sequence of the chimeric antigen receptor (CAR) is shown in SEQ ID NO: 8.

In the second aspect of the present invention, it provides a nucleic acid molecule encoding the chimeric antigen receptor according to the first aspect of the present invention.

In the third aspect of the present invention, it provides a vector which comprises the nucleic acid molecule according to the second aspect of the present invention.

In another preferred embodiment, the vector is selected from a group consisting of DNA, RNA, plasmid, lentiviral vector, adenoviral vector, retroviral vector, transposon and a combination thereof.

In another preferred embodiment, the vector is a lentiviral vector.

In another preferred embodiment, the vector is selected from a group consisting of pTomo lentiviral vector, plenti, pLVTH, pLJM1, pHCMV, pLBS.CAG, pHR, pLV, etc..

In another preferred embodiment, the vector includes a pTomo lentiviral vector.

In another preferred embodiment, the vector further comprises an element selected from a group consisting of: a promoter, a transcription enhancing element WPRE, a long terminal repeat sequence LTR, etc.

In the fourth aspect of the present invention, it provides a host cell comprising the vector according to the third aspect of the present invention, or expressing the CAR according to the first aspect of the present invention.

In the fifth aspect of the present invention, it provides an engineered immune cell comprising the vector according to the third aspect of the present invention, or expressing the CAR according to the first aspect of the present invention.

In another preferred embodiment, the engineered immune cell is a chimeric antigen receptor T cell (CAR-T cell), a chimeric antigen receptor NK cell (CAR-NK cell), a chimeric antigen receptor NKT cell (CAR-NKT cell) or a chimeric antigen receptor macrophage (CAR-M cell).

In another preferred embodiment, the engineered immune cell is a CAR-T cell.

In the sixth aspect of the present invention, it provides a method for the preparation of the engineered immune cell according to the fifth aspect of the present invention, which comprises a step of: transducing the nucleic acid molecule according to the second aspect of the present invention or the vector according to the third aspect of the present invention into an immune cell, thereby obtaining the engineered immune cell.

In another preferred embodiment, the method further comprises a step of detecting the function and effectiveness of the obtained engineered immune cell.

In the seventh aspect of the present invention, it provides a pharmaceutical composition comprising the CAR according to the first aspect of the present invention, the nucleic acid molecule according to the second aspect of the present invention, the vector according to the third aspect of the present invention, the host cell according to the fourth aspect of the present invention, and/or the engineered immune cell according to the fifth aspect of the present invention, and a pharmaceutically acceptable carrier, diluent or excipient.

In another preferred embodiment, the formulation is a liquid preparation.

In another preferred embodiment, the dosage form of the formulation is injection.

In another preferred embodiment, the concentration of the engineered immune cell in the formulation is $1\times10^3$-$1\times10^8$ cells/ml, and preferably $1\times10^4$-$1\times10^7$ cells/ml..

In the eighth aspect of the present invention, it provides a use of the CAR according to the first aspect of the present invention, the nucleic acid molecule according to the second aspect of the present invention, the vector according to the third aspect of the present invention, the host cell according to the fourth aspect of the present invention, and/or the engineered immune cell according to the fifth aspect of the present invention, in the preparation of a medicament or a formulation for preventing and/or treating a disease related to GrB receptor.

In another preferred embodiment, the disease related to GrB receptor includes a hematological tumor and solid tumor.

In another preferred embodiment, the hematological tumor is selected from a group consisting of: acute myeloid leukemia (AML), multiple myeloma (MM), chronic lymphocytic leukemia (CLL), acute lymphocytic leukemia (ALL), diffuse large B cell lymphoma (DLBCL) and a combination thereof.

In another preferred embodiment, the solid tumor is selected from a group consisting of: breast cancer, gastric cancer, hepatobiliary cancer, colorectal cancer, bladder cancer, non-small cell lung cancer, ovarian cancer and esophageal cancer, glioma, lung cancer, pancreatic cancer, prostate cancer, liver cancer and a combination thereof.

In another preferred embodiment, the disease related to GrB receptor is selected from a group consisting of: acute myeloid leukemia, breast cancer, endometrial cancer, and rectal cancer.

In another preferred embodiment, the disease related to GrB receptor is a tumor expressing a GrB receptor.

The disclosure also provides a use of the engineered immune cell according to the fifth aspect of the present invention or the pharmaceutical composition according to the seventh aspect of the present invention, in preventing and/or treating a tumor or cancer.

The disclosure also provides a method of treating a disease which comprises: administering an effective amount of the engineered immune cell according to the fifth aspect of the present invention, or the pharmaceutical composition of the seventh aspect of the present invention, to a subject in need thereof.

In another preferred embodiment, the disease is a cancer or tumor.

In another preferred embodiment, the engineered immune cell or the CAR immune cell comprised in the pharmaceutical composition is a cell derived from the subject (an autologous cell).

In another preferred embodiment, the engineered immune cell or the CAR immune cell comprised in the pharmaceutical composition is a cell derived from a healthy individual (an allogeneic cell).

The present disclosure provides a method for preparing a chimeric antigen receptor immune cell based on GrB and the application thereof.

The present disclosure provides GrB receptors, including but not limited to proteins such as HSP70, HSP60, HSP90 and other proteins that can bind to the GrB molecule.

The present disclosure provides a CAR structure comprising an extracellular GrB sequence specifically bound to a human GrB receptor, a hinge and transmembrane region, and an intracellular signal transduction region.

The present disclosure provides a chimeric antigen receptor immune cell, which includes but is not limited to T cells, NK cells, NKT cells, macrophages, etc.

The present disclosure also provides a treatment method that can be combined with other treatment methods such as chemotherapy, radiotherapy, targeted therapy, etc.

The present disclosure also provides the application of chimeric antigen receptor immune cells, and the treatment using the modified immune cells on GRB receptor related diseases including but not limited to tumors, aging, obesity, cardiovascular diseases, diabetes, neurodegenerative diseases, infectious diseases, etc.

**Description of Figure**

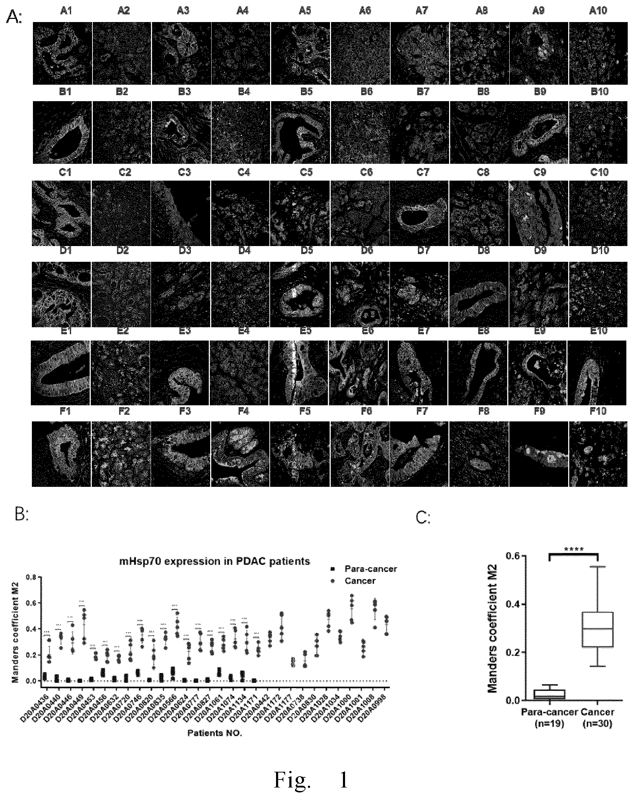

Figure 1 shows the co-localization analysis of HSP70 and β-catenin in pancreatic cancer tissue. A shows the immunohistochemical staining of HSP70 (red) and β-Catenin (green) in a pancreatic cancer tissue chip. A1-E6 represent the corresponding cancer tissue and paracancerous tissue, with odd numbers indicating paracancerous tissues and even numbers indicating cancer tissues. E7-F10 represent pancreatic cancer tissues. B shows the co-localization analysis of HSP70 and β-catenin in each cancer tissue and paracancerous tissue in the pancreatic cancer tissue chips. C shows the co-localization analysis of HSP70 and β-catenin in cancer tissues and paracancerous tissues.

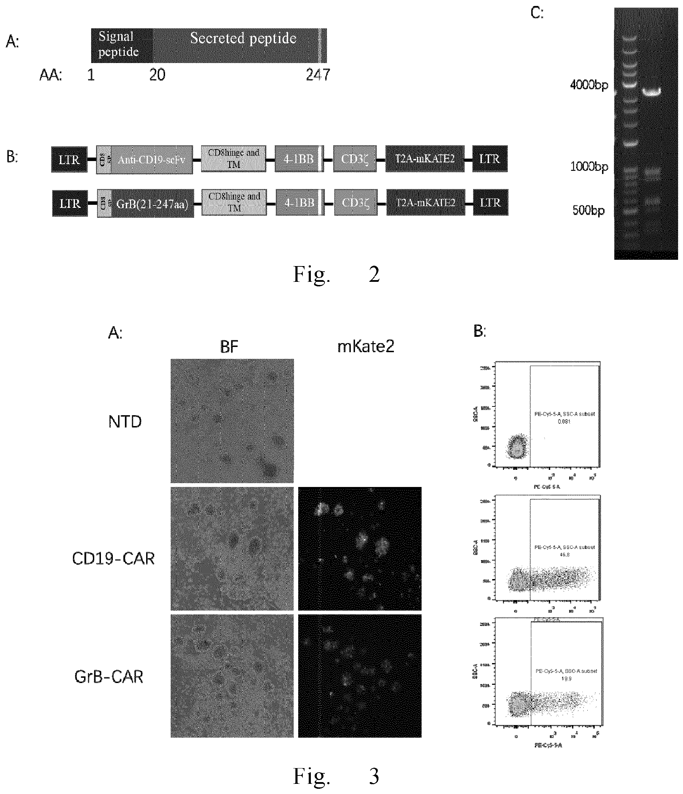

Figure 2 shows the construction of GrB-CAR vector: A shows the schematic diagram of GrB sequence, wherein 1-22 AA is the signal peptide, 21-247 AA is the mature polypeptide. B shows a schematic diagram of the control plasmid CD19-CAR and GrB-CAR, wherein the signal peptide, hinge region, and transmembrane region are derived from human CD8 molecules, 4-1BB is derived from human CD137, CD3ζ is derived from human CD3 and mKate2 is a fluorescent marker used for detecting CAR expression. C shows the identification of pTomo-GrB-CAR vector by HindIII enzyme digestion.

Figure 3 shows the detection of CAR transfection efficiency, A shows the cell fluorescence expression of T cells infected with CD19-CAR and GrB-CAR after 72 hours, where BF represents the bright field and mKate2 is fluorescence expression of CAR. B shows the fluorescence expression detected by flow cytometry.

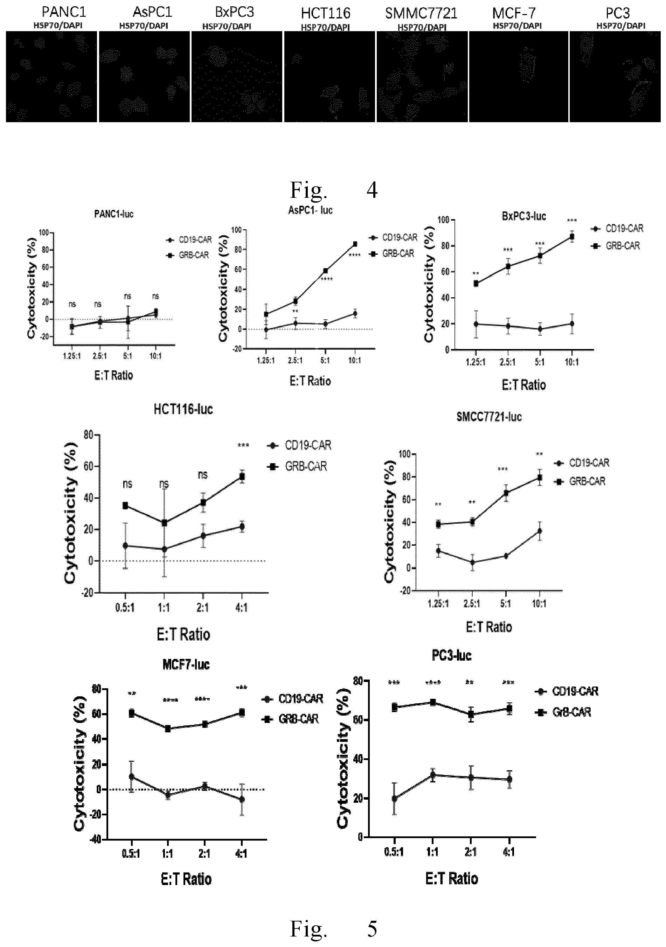

Figure 4 shows the mHSP70 expression in different tumor cell lines detected by immunofluorescence technology.

Figure 5 shows the *in vitro* killing test results of GrB-CAR on different tumor cell lines,

wherein, PANC1, BxPC3, AsPC1 are pancreatic cancer cell lines; PC3 is a prostate cancer cell line; SMMC7721 is a liver cancer cell line; HCT116 is a colorectal cancer cell line, and MCF7 is a breast cancer cell line.

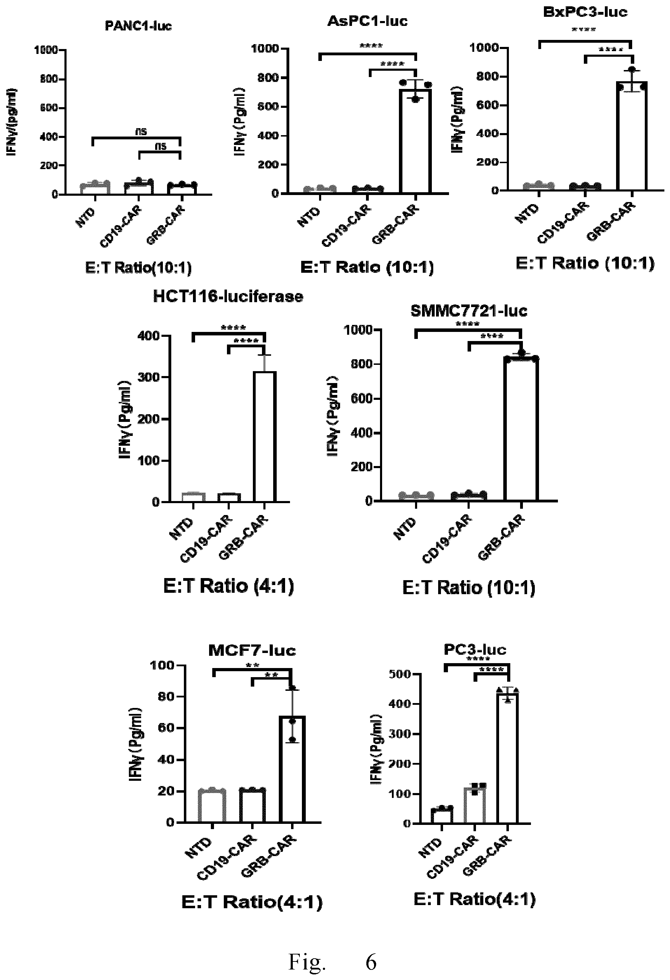

Figure 6 shows the detection result of IFNγ release.

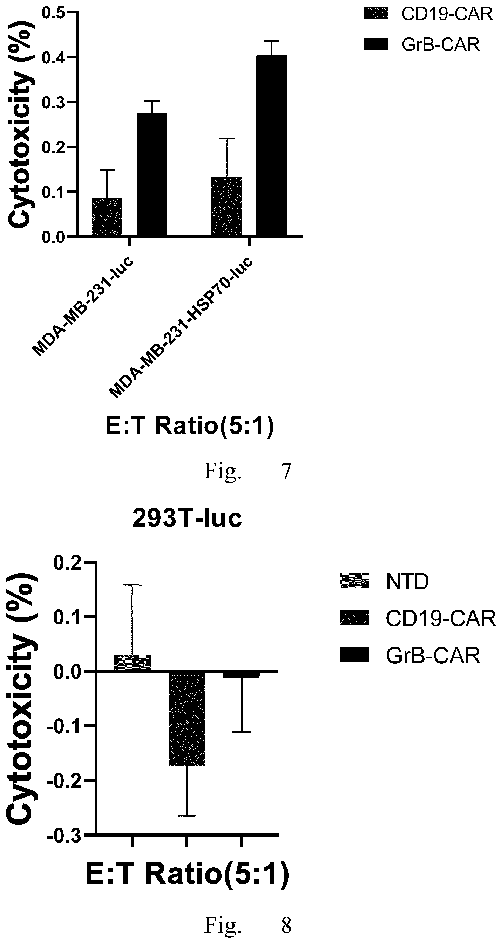

Figure 7 shows the effect of HSP70 overexpression on cell killing. MDA-MB231 cells are breast cancer cell lines.

Figure 8 shows the killing effect of GrB-CAR on normal cells.

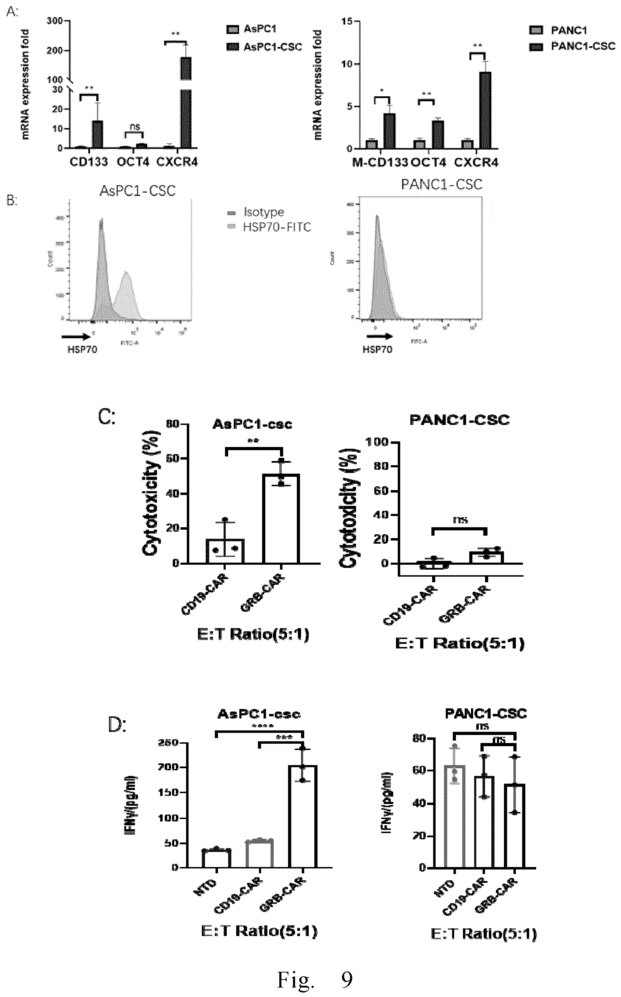

Figure 9 shows GrB-CART killing pancreatic cancer stem cells. A shows the expression detection of stem cell markers CD133, OCT4 and CXCR4 in AsPC1-CSC and PANC1-CSC. B shows the HSP70 expression in AsPC1-CSC and PANC1-CSC detected by flow cytometry. C and D show the killing of GrB-CART on AsPC1-CSC and PANC1-CSC (C) and the IFNγ release (D).

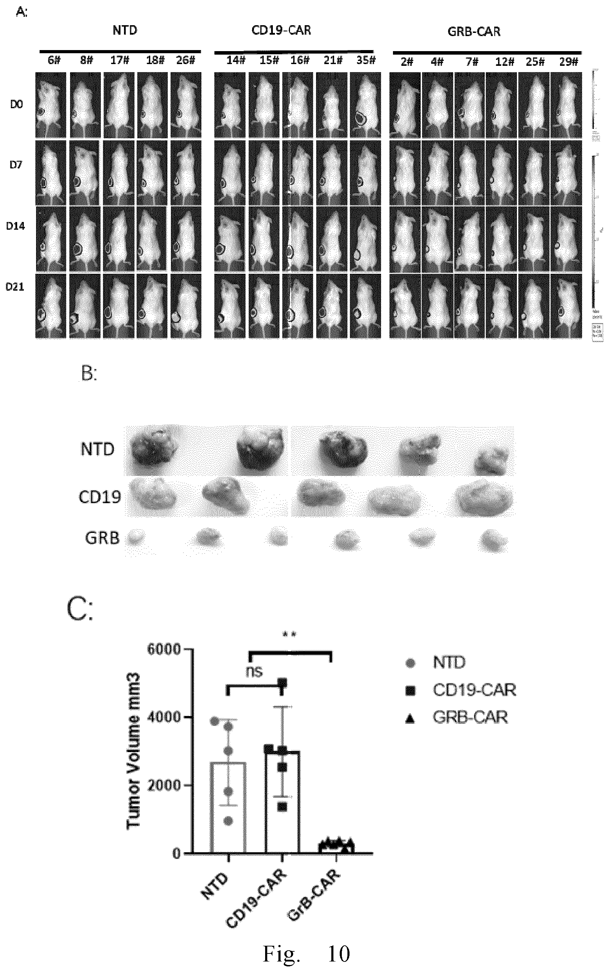

Figure 10 shows the inhibition by GrB-CART on AsPC1-luc xenograft tumors in nude mice. A and B show the growth of subcutaneous xenograft tumors at different time periods after GrB-CART reinfusion. The photograph (B) and volume measurement (C) of subcutaneous xenograft tumors after the experiment.

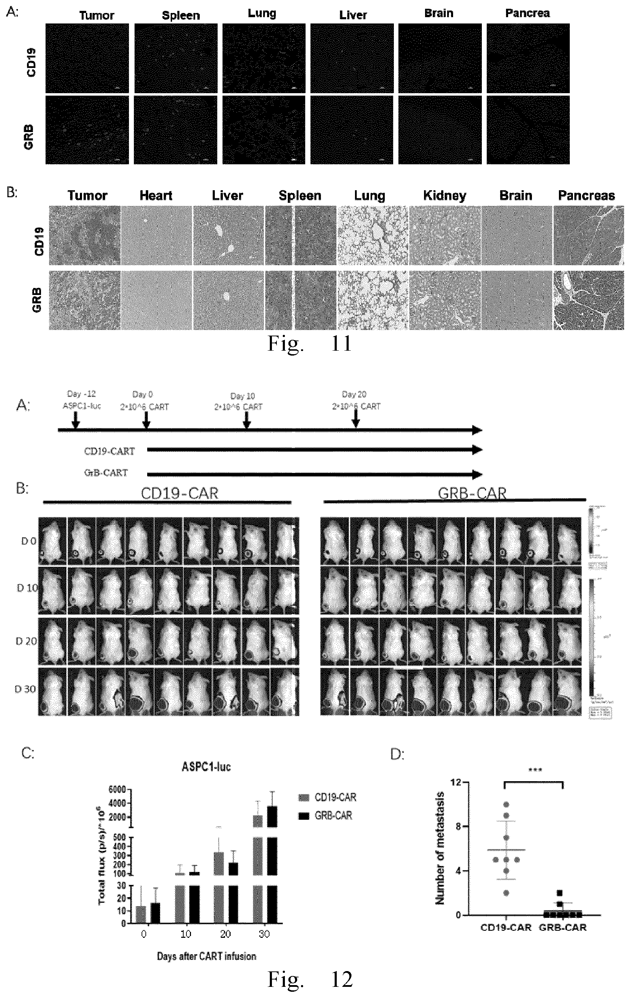

Figure 11 shows that GrB-CARTs enrich in subcutaneous xenograft tumors and do not cause damage to major organs. A shows the presence of T cells in subcutaneous xenograft tumors, liver, lung, pancreas, spleen, brain, etc. detected by immunohistochemistry. B shows HE staining of subcutaneous xenograft tumors, heart, liver, lung, spleen, kidney, pancreas, brain, etc. after CART reinfusion.

Figure 12 shows the inhibition of AsPC1 metastasis by GrB-CART. A is the experimental process. B and C show the growth (B) and quantification (C) of subcutaneous xenograft tumors at different time periods after CD19-CART and GrB-CART reinfusion. D shows the statistics of metastatic nodules in lung of the CD19-CART and GrB-CART groups after the experiment.

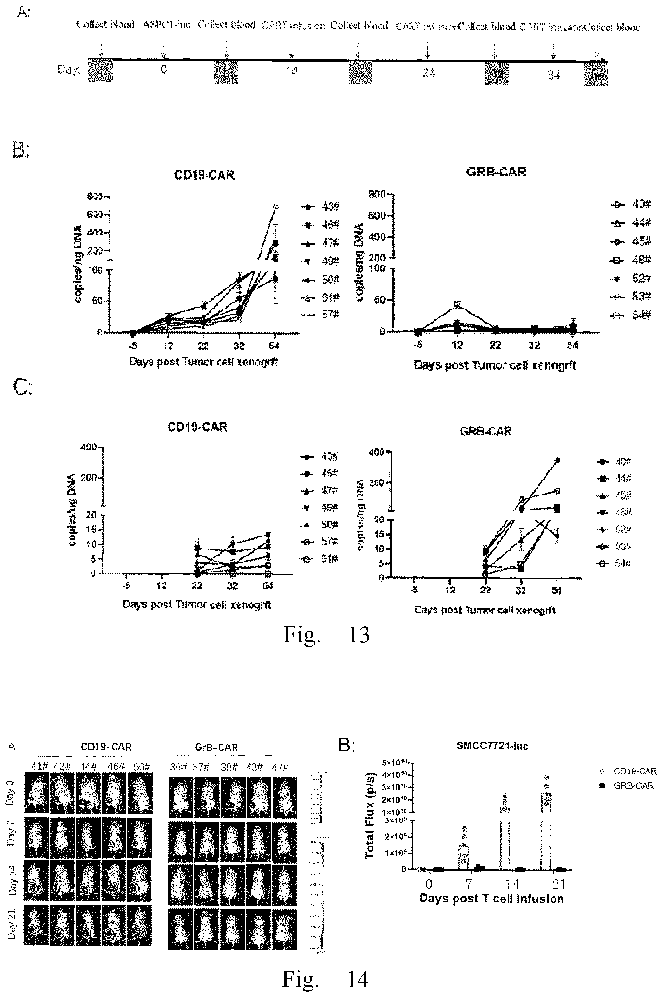

Figure 13 shows the inhibition of tumor cells in peripheral blood by GrB-CART. A shows a schematic diagram of the experimental blood collection process. B shows the detection of tumor cells in peripheral blood at different times after CD19-CART and GrB-CART reinfusion. C shows the detection of CART cells in peripheral blood at different times after CD19-CART and GrB-CART reinfusion.

Figure 14 shows the inhibition by GrB-CART on SMMC7721-luc xenograft tumors in nude mice. A shows the growth of subcutaneous xenograft tumors at different time periods after GrB-CART reinfusion. B shows the quantification of subcutaneous xenograft tumors at different time periods after GrB-CART reinfusion.

EMBODIMENTS

After extensive and intensive research and widely screening, the inventor has developed the preparation and application of a chimeric antigen receptor immune cell based on GrB for the first time. The experimental results show that the CAR-T targeting GrB receptor of the present disclosure has a significant killing effect on target cells, a broad-spectrum anti-tumor cell effect and the like. On this basis, the present invention has been completed.

Terms

To make it easier to understand the present invention, certain technical and scientific terms are specifically defined below. Unless otherwise clearly defined herein, all other technical and scientific terms used herein have meanings commonly understood by those skilled in the art in the field to which the present invention belongs.

Unless otherwise defined, all technical and scientific terms used herein have the same meaning as commonly understood by one of ordinary skill in the art to which this invention belongs. As used herein, when used in reference to a specifically recited value, the term "about" means that the value may vary by no more than 1% from the recited value. For example, as used herein, the expression "about 100" includes all values between 99 and 101 (e.g., 99.1, 99.2, 99.3, 99.4, etc.).

As used herein, the term "optional" or "optionally" means that the event or situation described thereafter may occur, but not necessarily.

As used herein, the terms "containing" or "comprising (including)" can be open, semi-closed, and closed. In other words, the term also includes "consisting essentially of," or "consisting of."

"Transduction", "transfection", "transformation" or the terms used herein refer to the process of delivering exogenous polynucleotides to host cells, and transcribing and translating to produce peptide products, including the use of plasmid molecules to introduce exogenous polynucleotides into host cells (such as E. coli).

"Gene expression" or "expression" refers to the process of gene transcription, translation, and post-translational modification to produce RNA or protein products of genes.

"Polynucleotide" refers to the polymerized forms of nucleotides of any length, including deoxyribonucleotides (DNA), ribonucleotides (RNA), and heterozygous sequences and analogues thereof. Polynucleotides can include modified nucleotides, such as methylated or capped nucleotides or nucleotide analogues. The term polynucleotides used herein refers to interchangeable single and double stranded molecules. Unless otherwise specified, polynucleotides in any embodiment described herein include double stranded form and two complementary single strands known or predictable to form a double stranded form.

The substitution of conservative amino acids is known in this field. In some embodiments, potential substituted amino acids are within one or more of the following groups: glycine, alanine; and valine, isoleucine, leucine, and proline; aspartate, glutamic acid; asparagine, glutamine; serine, threonine, lysine, arginine, and histidine; and/or phenylalanine, tryptophan, and tyrosine; methionine and cysteine. In addition, the present invention also provides non-conservative amino acid substitutions that allow amino acids from different functional groups to be substituted.

Those skilled in the art will easily understand the meaning of all parameters, sizes, materials, and configurations described herein. The actual parameters, sizes, materials, and configurations depend on the specific application described herein.

It should be understood that for any method described in this article that includes more than one step, the order of steps is not necessarily limited to the order described in these embodiments.

To make the disclosure easier to understand, some terms are firstly defined. As used in this application, unless expressly stated otherwise herein, each of the following terms shall have the meanings given below. Other definitions are set forth throughout the application.

The term "about" may refer to a value or composition within an acceptable error range for a particular value or composition as determined by those skilled in the art, which will depend in part on how the value or composition is measured or determined.

The term "administering" refers to the physical introduction of a product of the present invention into a subject using any one of various methods and delivery systems known to those skilled in the art, including intravenous, intramuscular, subcutaneous, intraperitoneal, spinal or other parenteral administration, such as by injection or infusion.

**Heat shock protein family**

Heat shock response is a self-protection mechanism produced by cells or tissues in the face of various stress stimuli, often accompanied by upregulation of heat shock proteins (HSPs) expression. According to molecular weight, heat shock proteins mainly include HSP100, HSP90, HSP70, HSP60, HSP40, HSP27, HSP26, etc. HSP70, as the most important heat shock protein in humans, is low expressed and localized within cells under normal physiological conditions, but overexpressed in tumor cells to promote cell proliferation, inhibit cell aging, and resist stress-induced cell apoptosis. Inhibiting HSP70 in tumor cells can lead to cell death and the formation of transplanted tumors in nude mouse. HSP70 is highly expressed in most tumor samples, which can be used as a molecular marker of early prostate cancer and liver cancer, and is related to the prognosis of various tumors such as acute myeloid leukemia, breast cancer, endometrial cancer and rectal cancer.

Methods targeting HSP70 expression, such as shRNA, CRISPR/Cas9 knockout, aptamers, inhibitors and the like are expected to be new approaches for cancer treatment. However, due to the complex heat shock protein family in eukaryotes, and the certain functional compensation among members of the heat shock protein family, other members of the HSPs family may compensate for the effect of HSP70 while the above methods reduce the expression of HSP70.

Part of the overexpressed HSP70 in tumor cells can be translocated to the cell membrane (mHSP70). The intracellular HSP70 promotes the occurrence and development of tumors by maintaining tumor cell homeostasis, stabilizing cyclin D1, and inhibiting apoptosis caused by oncogenes, while the HSP70 on the cell membrane surface can be recognized by immune cells. Tumor cell lines with high expression of membrane HSP70 (mHSP70) can be killed by CD56bright/CD94+NK cells in the presence of pro-inflammatory cytokines. In the field of tumor immunotherapy, T cells, antigen-presenting cells (APCs), NK cells, etc. can exert anti-tumor effects through mHSP70. In addition, mHSP70 activates dendritic cells by binding to TRL4. Compared with mHSP70 negative tumor cells, tumor cells with high expression of mHSP70 are more likely to be killed by IL-2-activated NK cells. In 2015, a phase II clinical trial was conducted to investigate the effects of TKD and IL-2 activated NK cells on non-small cell lung cancer patients. The above results indicate that mHSP70 is an important target molecule for tumor treatment.

**Granzyme B (GrB)**

GrB is a serine protease released by cytotoxic T cells and natural killer (NK) cells, which can induce programmed cell death in target cells. Experimental data confirming that GrB can specifically bind to the extracellular domain of mHSP70, and enter the cytoplasm of target cells. GrB can target substrates in the cytoplasm and nucleus, and induce apoptosis through various pathways. GrB, as a multifunctional protease, may play an important role in various inflammatory or degenerative pathologies. Besides, an increasing number of studies have confirmed the important role of GrB in chronic inflammatory and autoimmune diseases.

In summary, cells (including but not limited to tumor cells) under stress may express higher level of HSP70 and causes its partial translocation to the cell membrane surface (mHSP70). GrB can specifically bind to mHSP70, but there is currently no CAR-T therapy technology constructed based on GrB.

On this basis, the present disclosure for the first time constructs GrB CART cells that can target GrB receptors (including but not limited to mHSP70) for the treatment of related diseases.

Chimeric antigen receptor (CAR) of the present invention

Chimeric antigen receptor (CAR) is composed of extracellular antigen recognition region, transmembrane region, and intracellular co-stimulatory signal region.

The design of CARs has gone through the following process. The first generation CAR has only one intracellular signal component, CD3ζ or FcγRI molecule. Because there is only one activation domain in the cell, it can only cause transient T cell proliferation and less cytokine secretion, and does not provide long-term T cell proliferation signals and sustained antitumor effects in vivo. Therefore, it has not achieved very good clinical efficacy. In the second generation CAR, based on the original structure, a co-stimulatory molecule such as CD28, 4-1BB, OX40 and ICOS is introduced. Compared with the first generation CAR, the function has been greatly improved, and the sustainability of CAR-T cells and the ability to kill tumor cells are further enhanced. Based on the second generation CAR, some new immune stimulatory molecules such as CD27 and CD134 are linked in tandem to develop the third and fourth generation CARs.

The extracellular segment of CAR can recognize a specific antigen, then the signal is transduced through the intracellular domain to induce cell activation, proliferation, cytotoxicity, and secretion of cytokines, thereby clearing target cells. Firstly, the patient's autologous cells (or cells from heterologous donors) are isolated, activated and genetically modified to produce CAR immune cells, and then injected into the same patient's body. This method has an extremely low probability of developing graft-versus-host disease, and the antigen is recognized by immune cells in a non-MHC restricted manner.

CAR immunotherapy has achieved a very high clinical response rate in the treatment of hematological malignancies, which is unmatched by any previous treatment method and has sparked a wave of clinical research worldwide.

Specifically, the chimeric antigen receptor (CAR) of the present invention comprises an extracellular domain, a transmembrane domain, and an intracellular domain.

The extracellular domain comprises a target-specific binding element. The extracellular domain can be an ScFv based on antigen-antibody specific binding, or a natural sequence based on ligand-receptor specific binding or a derivative thereof.

In the present disclosure the extracellular domain of the chimeric antigen receptor can specifically bind to a GrB receptor. More preferably, the extracellular binding domain of the chimeric antigen receptor of the present invention has an amino acid sequence of positions 21-247 of the sequence shown in SEQ ID NO: 1.

The intracellular domain includes a co-stimulatory signaling region and a $\zeta$ chain. The co-stimulatory signaling region refers to a part of the intracellular domain that includes a co-stimulatory molecule. The co-stimulatory molecule is a cell surface molecule required for efficient response of lymphocytes to antigens, rather than a antigen receptor or its ligand.

A linker (or linking peptide) can be incorporated between the extracellular domain and the transmembrane domain of the CAR, or between the cytoplasmic domain and the transmembrane domain of the CAR. As used herein, the term "linker" generally refers to any oligopeptide or polypeptide that plays a role of linking the transmembrane domain to the extracellular domain or the cytoplasmic domain in a polypeptide chain. The linker may comprise 0-300 amino acids, preferably 2-100 amino acids and most preferably 3-50 amino acids.

When the CAR of the present invention is expressed in T cell, antigen recognition can be performed based on antigen binding specificity. When the CAR binds to its associated antigen, it affects tumor cell, causing tumor cell to fail to grow, to death or to be affected otherwise, causing the patient's tumor burden to shrink or eliminate. The antigen binding domain is preferably fused to one or more intracellular domains of the co-stimulatory molecule and the $\zeta$ chain. Preferably, the antigen binding domain is fused with an intracellular domain of a combination of an ICOS and/or 4-1BB signaling domain and a CD3$\zeta$ signaling domain.

As for the hinge region and the transmembrane region (transmembrane domain), the CAR can be designed to comprise a transmembrane domain fused to the extracellular domain of the CAR. In one embodiment, a transmembrane domain that is naturally associated with one of the domains in the CAR is used. In some embodiments, transmembrane domains may be selected or modified by amino acid substitutions to avoid binding such domains to the transmembrane domain of the same or different surface membrane proteins, thereby minimizing the interaction with other members of the receptor complexes.

The intracellular domain in the CAR of the present disclosure comprises the 4-1BB co-stimulatory domain and the signaling domain of CD3$\zeta$.

In one embodiment of the present invention, the CAR can specifically target mHSP70.

## Chimeric antigen receptor immune cell (CAR-immune cell)

In the present disclosure it provides a chimeric antigen receptor immune cell comprising a chimeric antigen receptor of the present invention that specifically targets a GrB receptor (preferably mHSP70).

The chimeric antigen receptor immune cell of the present invention can be a CAR-T cell, a CAR-NK cell, a CAR-NKT cell, or a CAR-M cell. Preferably, the chimeric antigen receptor immune cell of the present invention is a CAR-T cell.

As used herein, the terms "CAR-T cell", "CAR-T", and "CAR-T cell of the present invention" all refer to the CAR-T cell described in the fifth aspect of the present invention.

CAR-T cells have the following advantages over other T cell-based therapies: (1) the action process of CAR-T cells is not limited by MHC; (2) given that many tumor cells express the same tumor markers, a CAR gene targeting a particular tumor marker, once being completely constructed, can be widely utilized; (3) CARs can utilize both tumor protein markers and non-protein markers of carbohydrates and lipids, expanding the target range of tumor markers; (4) the use of patient autologous cells reduces the risk of rejection reactions; (5) CAR-T cells have immune memory function and can survive in the body for a long time.

As used herein, the terms "CAR-NK cell", "CAR-NK", and "CAR-NK cell of the present invention" all refer to the CAR-NK cell described in the fifth aspect of the present invention. The CAR-NK cells of the present disclosure can be used in treating tumors with high expression of heat shock protein (preferably mHSP70).

Natural killer (NK) cells are a major type of immune effector cells that protect the body from viral infections and tumor cell invasion through non antigen-specific pathways. Engineered (genetic modified) NK cells may acquire new functions, including the ability to specifically recognize tumor antigens and enhanced anti-tumor cytotoxicity.

Compared with autologous CAR-T cells, CAR-NK cells also have advantages such as: (1) directly killing tumor cells by releasing perforin and granzyme, without killing normal cells in the body; (2) releasing a small amount of cytokines, thereby reducing the risk of cytokine storms; (3) extremely easiness to be expanded and developed into "ready-made" products *in vitro.* In addition, it is similar to CAR-T cell therapy.

## Vector

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically.

The present invention also provides vectors comprising the nucleic acid molecule of the present invention. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells, such as hepatocytes. They also have the advantage of low immunogenicity.

In brief, the expression cassette or nucleic acid sequence of the present disclosure is typically and operably linked to a promoter, and incorporated into an expression vector. The vectors can be suitable for replication and integration in eukaryotes. Typical cloning vectors contain transcription and translation terminators, initiation sequences, and promoters useful for regulation of the expression of the desired nucleic acid sequence.

The expression constructs of the present disclosure may also be used for nucleic acid immune and gene therapy, using standard gene delivery protocols. Methods for gene delivery are known in the art. See, e.g., U.S, Pat. Nos. 5,399,346, 5,580,859, and 5,589,466,

The nucleic acid can be cloned into various vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al, (2001 , Molecular Cloning: A Laboratory Manual, Cold Spring Harbor Laboratory, New York), and in other virology and molecular biology manuals. Viruses which are useful as vectors include but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326, 193).

A number of viral based systems have been developed for transferring a gene into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1$\alpha$ (EF-1$\alpha$). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the present invention should not be limited to the use of constitutive promoters. Inducible promoters are also contemplated as part of the present invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionein promoter, a glucocorticoid promoter, a progesterone promoter, and a tetracycline promoter.

In order to assess the expression of a CAR polypeptide or portions thereof, the expression vector to be introduced into a ceil can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). In one embodiment the reporter gene is a gene encoding the mKate2 red fluorescent protein. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat, Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

In a preferred embodiment of the present invention, the vector is a lentiviral vector.

**Formulation**

The present disclosure provides a formulation (or preparation) comprising the chimeric antigen receptor (CAR) according to the first aspect of the present invention, the nucleic acid molecule according to the second aspect of the present invention, the vector according to the third aspect of the present invention, or the host cell according to the fourth aspect of the present invention or the engineered immune cell according to the fifth aspect of the present invention, and a pharmaceutically acceptable carrier, diluent or excipient. In one embodiment, the formulation is a liquid preparation. Preferably, the formulation is an injection. Preferably, the concentration of the CAR-T cells in the formulation is $1 \times 10^3$-$1 \times 10^8$ cells/ml, more preferably $1 \times 10^4$-$1 \times 10^7$ cells/ml.

In one embodiment, the formulation may comprises buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. The formulation of the present disclosure is preferably formulated for intravenous administration.

**Therapeutic application**

The present disclosure comprises therapeutic applications by using cells (e.g., T cells) transduced with a lentiviral vector (LV) encoding the expression cassette of the present disclosure The transduced T cells target a GrB receptor (preferably mHSP70), synergistically activate immune cells, and cause T cell immune responses, thereby significantly increasing the killing efficiency against tumor cells.

Therefore, the present disclosure also provides a method for stimulating a T cell-mediated immune response to a target cell population or tissue in a mammal comprising a step of administering to the mammal a CAR cell of the present disclosure

In one embodiment, the present disclosure comprises a cell therapy, wherein T cells from autologous patient (or heterologous donor) are isolated, activated and genetically modified to generate CAR-T cells, and then injected into the same patient. The probability of graft-versus-host-disease in the way is extremely low, and antigens are recognized by T cells in a non-MHC-restricted manner. In addition, one CAR-T can treat all cancers that express the antigen. Unlike antibody therapy, CAR-T cells are able to replicate in vivo and result in long-term persistence that can lead to sustained tumor control.

In one embodiment, the CAR-T cells of the present disclosure can undergo robust in vivo T cell expansion and can persist for an extended period. In addition, the CAR mediated immune response may be part of an adoptive immunotherapy approach in which CAR-modified T cells induce an immune response specific to the antigen binding moiety in the CAR. For example, an anti-GrB receptor (preferably mHSP70) CAR-T cell elicits an immune response specific against cells expressing GrB receptor (preferably mHSP70).

Cancers that may be treated include tumors that are unvascularized or largely unvascularized, and tumors that are vascularized. Cancers may include non-solid tumors (such as hematological tumors, for example, leukemias and lymphomas) or solid tumors. Types of cancers to be treated with the CARs of the present invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/-cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblasts, promyeiocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors (such as sarcomas and carcinomas) include fibrosarcoma, myxosarcoma, liposarcoma, mesothelioma, malignant lymphoma, pancreatic cancer, ovarian cancer, breast cancer, gastric cancer, hepatobiliary cancer, colorectal cancer, bladder cancer, non-small cell lung cancer, ovarian and esophageal cancer, glioblastoma, and lung cancer.

The CAR-modified T cells of the present disclosure may also serve as a type of vaccine for ex vivo immunization and/or in vivo therapy in a mammal. Preferably, the mammal is a human.

With respect to ex vivo immunization, at least one of the following occurs in vitro prior to administering the cells into a mammal: i) expanding the cells, ii) introducing a nucleic acid encoding a CAR to the cells, and/or iii) cryopreservation of the cells.

Ex vivo procedures are well known in the art and are discussed more fully below. Briefly, cells are isolated from a mammal (preferably a human) and genetically modified (i.e., transduced or transfected in vitro) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of ex vivo immunization, the present disclosure also provides compositions and methods for in vivo immunization to elicit an immune response directed against an antigen in a patient.

The present disclosure provides a method for treating tumors comprising administering to a subject in need thereof, a therapeutically effective amount of the CAR-modified T cells of the present disclosure

The CAR-modified T cells of the present invention may be administered either alone, or as a pharmaceutical composition in combination with diluents and/or with other components such as IL-2, IL-17 or other cytokines or cell populations. Briefly, pharmaceutical compositions of the present disclosure may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present disclosure are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an effective amount", "an immunologically effective amount", "an anti-tumor effective amount", "an tumor-inhibiting effective amount", or "a therapeutic amount" is indicated, the precise amount of the compositions of the present disclosure to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of $10^4$ to $10^9$ cells/kg body weight, preferably $10^5$ to $10^6$ cells/kg body weight, including all integer values within those ranges. T cell compositions may also be administered multiple times at these dosages. The cells can be administered by using infusion techniques that are commonly known in immunotherapy (see, e.g., Rosenberg et al,, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (i.v.) injection, or intraperitoneally. In one embodiment, the T cell compositions of the present disclosure are administered to a patient by intradermal or subcutaneous injection. In another embodiment, the T cell compositions of the present disclosure are preferably administered by i.v. injection. The compositions of T cells may be injected directly into a tumor, lymph node, or site of infection.

In certain embodiments of the present disclosure cells activated and expanded using the methods described herein, or other methods known in the art where T cells are expanded to therapeutic levels, are administered to a patient in conjunction with (e.g., before, simultaneously or following) any number of relevant treatments, including but not limited to treatment with agents such as antiviral therapy, cidofovir and interleukin-2, Cytarabine (also known as ARA-C) or natalizumab treatment for MS patients or efalizumab treatment for psoriasis patients or other treatments for specific tumor patients. In further embodiments, the T cells of the present disclosure may be used in combination with chemotherapy, radiation, immunosuppressive agents, such as cyclosporin, azathioprine, methotrexate, mycophenolate, and FK506, antibodies, or other immunotherapeutic agents. In a further embodiment, the cell compositions of the present disclosure are administered to a patient in conjunction with (e.g., before, simultaneously or following) bone marrow transplantation, or the use of chemotherapy agents such as, fludarabine, external-beam radiation therapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to practices in the field. In general, $1 \times 10^6$ to $1 \times 10^{10}$ of CAR-T cells of the present disclosure can be applied to patients by means of, for example, intravenous infusion in each treatment or each course of treatment.

**The main advantages of the present invention include:**

1) Target specificity: mHSP70 is only GrB receptors that have been currently reported, so the target specificity of the present CAR is high.
2) High specificity to target cells: taking mHSP70 as an example, it is not expressed on the cell membrane of normal cells, but can translocate to the cell membrane under pressure stress (such as in tumors). Therefore, the present CAR only targets malignant cells with high expression of HSP70 on the cell membrane and has no killing effect on normal cells.
3) The present invention utilizes a ligand-receptor binding mechanism instead of the traditional scfv. Safety tests in animals, especially primates, better reflect their safety in the human body.

The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

Table A. Summary of amino acid sequences involved in the present invention

| Name of sequence | Sequence | SEQ ID NO: |
|---|---|---|
| Full-length GrB protein | MQPILLLLAFLLLPRADAGEIIGGHEAKPHSRPYMAYLMIWDQK SLKRCGGFLIRDDFVLTAAHCWGSSINVTLGAHNIKEQEPTQQF IPVKRPIPHPAYNPKNFSNDIMLLQLERKAKRTRAVQPLRLPSN KAQVKPGQTCSVAGWGQTAPLGKHSHTLQEVKMTVQEDRKC ESDLRHYYDSTIELCVGDPEIKKTSFKGDSGGPLVCNKVAQGIV SYGRNNGMPPRACTKVSSFVHWIKKTMKRY | 1 |
| mKate2 red fluorescent protein | MSELIKENMHMKLYMEGTVNNHHFKCTSEGEGKPYEGTQT MRIKAVEGGPLPFAFDILATSFMYGSKTFINHTQGIPDFFKQS FPEGFTWERVTTYEDGGVLTATQDTSLQDGCLIYNVKIRGV NFPSNGPVMQKKTLGWEASTETLYPADGGLEGRADMALKL VGGGHLICNLKTTYRSKKPAKNLKMPGVYYVDRRLERIKE ADKETYVEQHEVAVARYCDLPSKLGHKLN | 2 |
| Signal peptide | MAAATGPSFWLGNETLKVPLAL | 3 |
| CD8 hinge region | TTTPAPRPPTPAPTIASQPLSLRPEACRPAAGGAVHTRGLDF ACD | 4 |
| CD28 transmembrane region | IYIWAPLAGTCGVLLLSLVITLYC | 5 |
| Co-stimulat ory signaling molecule derived from 4-1BB | KRGRKKLLYIFKQPFMRPVQTTQEEDGCSCRFPEEEEGGCE LR | 6 |
| Cytoplasmic signal transduction sequence derived from CD3ζ | VKFSRSADAPAYKQGQNQLYNELNLGRREEYDVLDKRRGR DPEMGGKPRRKNPQEGLYNELQKDKMAEAYSEIGMKGERR RGKGHDGLYQGLSTATKDTYDALHMQALPPR | 7 |

14

| Name of sequence | Sequence | SEQ ID NO: |
|---|---|---|
| Preferred full-length CAR of the present invention | MALPVTALLLPLALLLHAARPIIGGHEAKPHSRPYMAYLMI WDQKSLKRCGGFLIRDDFVLTAAHCWGSSINVTLGAHNIKE QEPTQQFIPVKRPIPHPAYNPKNFSNDIMLLQLERKAKRTRA VQPLRLPSNKAQVKPGQTCSVAGWGQTAPLGKHSHTLQEV KMTVQEDRKCESDLRHYYDSTIELCVGDPEIKKTSFKGDSG GPLVCNKVAQGIVSYGRNNGMPPRACTKVSSFVHWIKKTM KRYTTTPAPRPPTPAPTIASQPLSLRPEACRPAAGGAVHTRG LDFACDIYIWAPLAGTCGVLLLSLVITLYCKRGRKKLLYIFK QPFMRPVQTTQEEDGCSCRFPEEEEGGCELRVKFSRSADAP AYKQGQNQLYNELNLGRREEYDVLDKRRGRDPEMGGKPR RKNPQEGLYNELQKDKMAEAYSEIGMKGERRRGKGHDGL YQGLSTATKDTYDALHMQALPPRGSG | 8 |

EP 4 357 369 B1

## Example 1: Detection of HSP70 Localization in Pancreatic Cancer Sample Chip

A pancreatic cancer sample microarray was purchased from Shanghai Outdo Biotech, containing 23 pairs of adjacent non-cancerous and cancerous tissue samples, as well as 14 cases of pancreatic cancer samples. Immunohistochemical staining was used to detect the expression of HSP70 (red) and the cell membrane marker β-catenin (green). Laser confocal microscopy was used to capture images and IMAGE J software was utilized to analyze the co-localization of the two markers.

**Result:** The results are shown in Fig. 1. The results demonstrates that, compared to adjacent non-cancerous tissues, the co-localization ratio of HSP70 and β-catenin is higher in cancerous tissues, indicating that HSP70 is localized on the cell membrane in cancerous tissues.

## Example 2: Preparation of GrB-CAR Vector

The nucleotide sequence of GrB (NM_004131.6) and gene sequence information of human CD8α hinge region, human CD8 transmembrane region, human 4-1BB intracellular region, and human CD3ζ intracellular region were obtained from the NCBI database. The CD8 signal peptide and the extracellular domain of GrB were synthesized by BGI, and the nucleotide sequence of the CAR molecule was digested with XbaI (Thermo) and NheI (Thermo), ligated by T4 DNA ligase (NEB) and inserted into the lentiviral vector pTomo, which had already been inserted with CD8TM, 4-1BB, and CD3ζ. The vector was transformed into competent E. coli (Stbl3).

The recombinant plasmid was sent to TsingKe Biotechnology Co., Ltd. for sequencing, and the sequencing results were aligned to confirm the correctness of the plasmid. The sequencing primer used was the universal sequencing primer CMV-F: CGCAAATGGGCGGTAGGCGTG.

All plasmids were extracted using the endotoxin-free large-scale extraction kit from QIAGEN. The purified plasmids were used to transfect 293T cells with Beyotime lipo6000 for lentivirus packaging.

The results of this example are shown in Fig. 2.

## Example 3: Packaging Virus

Packaging was performed in a 15 cm dish using 293T cells less than 20 passages old. Transfection was carried out when the confluence of 293T cells was about 80%-90%. A plasmid mixture dissolved in 2ml OPTIMEM was prepared (core plasmid 20ug, pCMVΔR8.9 10ug, PMD2.G 4ug); and 2ml OPTIMEM and 68ul of lipo 6000 were combined in another tube. After set at room temperature for 5 minutes, the plasmid complex was added to the liposome complex and set at room temperature for 20 minutes. The mixture was then added dropwise to the 293T cells and incubated at 37°C for 6 hours before removing the medium. Pre-warmed complete media were added. Viral supernatants were collected at 48 hours and 72 hours post-transfection, centrifuged at 3000 rpm for 20 minutes at 4°C, filtered through a 0.45um filter membrane, and then concentrated by centrifugation at 25000rpm for 2.5 hours at 4°C. The concentrated virus was dissolved overnight in 30 ul of virus dissolving solution, and the virus titer was detected by QPCR.

## Example 4: Preparation of CART cells

Mononuclear cells were isolated from human peripheral blood using Ficoll separation solution, and purified CD3+ T cells were obtained using RosetteSep Human T Cell Enrichment Cocktail (Stemcell technologies). T cells were activated with CD3/CD28 magnetic beads (Life technology) and stimulated and cultured with 200U/ml of IL2 (PeproTech) added for 48 hours before viral infection. Lentivirus was used to infected T cells at an MOI=20 in the presence of lentiboost to prepare CART cells. The medium was replaced after one-day infection.

## Example 5: Detection of Positive Rate of CART Cells Infection by Flow Cytometry

CART cells and NT cells (control group) were centrifuged and collected separately after 72 hours of virus infection. They were then washed once with PBS, and the supernatant was discarded. The cells were resuspended in PBS containing 2% FBS, and the positive rate was detected by flow cytometry.

The results of this example are shown in Fig. 3. The results indicate that the positive rate of CART cells is approximately 20%.

## Example 6: Detection of mHSP70 Expression in Various Target Cells

Target cells were seeded onto round slips of a 24-well plate and washed three times with PBST for 5 minutes each after 24 hours. They were then incubated with an antibody specific recognizing mHSP70 at room temperature for 1 hour.

The cells were fixed with 4% PFA for 20 minutes and blocked with 10% goat serum at room temperature for 1 hour.

The following day, the cells were washed three times with PBST for 5 minutes each. Cells were added with a secondary antibody that specific recognizes mHSP70 and is labeled with CY3, and incubated at room temperature for 1 hour. The cells were washed three times with PBS, and the nuclei were stained with DAPI. Confocal microscopy imaging was performed.

The results of this example are shown in Fig. 4. The results indicate that HSP70 is located on the cell membrane in pancreatic cancer cell lines AsPC1 and BxPC3, as well as liver cancer cell line SMMC-7721, colorectal cancer cell line HCT116, breast cancer cell line MCF-7 and prostate cancer cell line PC3, while pancreatic cancer cell line PANC1 does not express HSP70.

## Example 7: Construction of Luciferase-Expressing Target Cells

Luciferase fragment was amplified by PCR from the pGL3 -luciferase plasmid, and then ligated into the pTomo vector using XbaI and BamHI to construct the pTomo-luciferase plasmid. Fragments of IRES and puromycin were amplified from the pTomo and PLkO.1 plasmids, respectively. The pTomo-luciferase-IRES-Puro plasmid was successfully constructed by ligating these three fragments.

The lentiviral packaging and titration detection were performed as described above. The virus was used to infect human embryonic kidney 293T cells, human breast cancer cell line MCF7 and MDA-MB-231, prostate cancer cell line PC3, colorectal cancer cell line HCT116, liver cancer cell line SMMC7721, pancreatic cancer cell lines BxPc3, PANC1, and AsPC1.

After 48 hours, the cells were selected with puromycin (1 ug/ml) for one week to obtain 293T-luciferase, MCF7-luciferase, MDA-MB231-luciferase, PC3-luciferase, SMMC7721-luciferase, HCT116-luciferase, PANC1-luciferase, Bxpc3-luciferase, and AsPC1-luciferase cells.

## Example 8: Detection of CART Cells Killing

Luciferase-carrying cells were digested and counted, and the cell density was adjusted to 2*10^4/ml. 100ul of luciferase-carrying cells were seeded into a black 96-well plate, and CART/NT cells were adjusted to a density of 1*10^5 cells. They were then seeded into a black 96-well plate at different effector to target (E:T) ratios of 0.5:1, 1:1, 2:1, and 4:1, in a volume of 100 ul per well. The above target cells and T cells were mixed and incubated in a culture incubator for 24 hours. The supernatant was collected and stored at -80°C for the detection of IFNγ release. Cell killing was detected using the Promega fluorescence detection kit. First, the cells were lysed with 20 ul of 1*PLB lysis buffer for 20 minutes. Then, 100 ul of substrate was added to each well, and the reaction was immediately detected using a BioTek plate reader.

The cytotoxicity killing rate % = (1 - the fluorescence value of target cells with effector cells / the fluorescence value of target cells without effector cells) * 100%

The results are shown in Fig. 5. The results show that GrB-CAR can kill HSP70-expressing tumor cells dose-dependently.

## Example 9: Detection of IFNγ Cytokine Release

After being thawed from -80°C, the cell supernatant was used to detect IFNγ using the IFN gamma Human ELISA Kit (Life Technology).

The standard samples were dissolved using Standard Dilution Buffer, and gradiently diluted into standard samples of 1000 pg/ml, 500 pg/ml, 250 pg/ml, 125 pg/ml, 62.5 pg/ml, 31.2 pg/ml, 15.6 pg/ml, and 0 pg/ml.

To each well, 50 ul Incubation buffer, 50 ul sample to be detected and 50 ul IFNγ biotin conjugate solution were added, mixed well and incubated at room temperature for 90 minutes.

Then the following steps are performed in order:

(1) Wash the wells four times with 1* Wash Buffer, leaving each time for one minute.
(2) Add 100 ul 1* Streptavidin-HRP solution to each well and incubate at room temperature for 45 minutes.
(3) Wash the wells four times with 1* Wash Buffer, leaving each time for one minute.
(4) Add 100 ul Stabilized chromogen and incubate at room temperature for 30 minutes.
(5) Add 100 ul Stop solution to each well and mix well.
(6) Detect the absorbance at 450 nm.

The results are shown in Fig. 6. The results indicate that the killing effect of GrB-CART cells on PC3 cell line is corresponding to the release of IFNγ.

## Example 10: Effect of Overexpression of HSP70 on GrB-CART Killing Effect

The HSP1A1 nucleotide sequence was synthesized by BGI and linked by enzyme cleavage to construct a lentiviral vector pTomo-CMV-HSP70-IRES-Luciferase-T2A-Puro. The lentivirus was packaged as described previously and transfected into the MDAMB-231 cell line. After 10 days of Puromycin (1ug/ml) screening, stable screened cell lines were obtained, and killing experiments were conducted according to an effective-target ratio of 5:1. The steps for killing detection are the same as those in Example 8.

The results are shown in Fig. 7. The results show that GrB-CAR has significant killing effect on breast cancer cells.

In addition, the killing activity of GrB-CAR on normal cells (human embryonic kidney cell 293T) was detected using the same method.

The results are shown in Fig. 8. The results show that GrB-CAR has almost no killing activity on normal cells, which indicates that the GrB-CAR of the present invention has high safety performance.

## Example 11: Killing of GrB-CART on Pancreatic Cancer Stem Cells

Tumor cell lines AsPC1-luc and PANC1-luc were cultured in selective medium DMEM/F12 + EGF (20 ng/ml) + bFGF (20 ng/ml) + B27 (1×) + 1 % PS. After the tumor stem cells formed spheres, flow cytometry was used to detect HSP70 expression, and RNA was extracted and reverse transcribed into cDNA to detect the expression of tumor stem cell markers such as CD133, OCT4, and CXCR4. The detection of killing of tumor stem cells and cytokine release by GrB-CART were carried out as described previously.

The results shown in Fig. 7 indicate that compared to AsPC1 cells, the expression of stem cell markers CD133 and CXCR4 in AsPC1-CSC significantly increases; and compared to PANC1 cells, the expression of stem cell markers CD133, OCT4, and CXCR4 in PANC1-CSC significantly increases. Moreover, AsPC1-CSC expresses hsp70, while PANC1-CSC does not express hsp70. GrB-CART significantly kills AsPC1-CSC cells but does not kill PANC1-CSC cells, and the killing effect is positively correlated with IFNγ release.

## Example 12: Inhibiting Effect of GrB-CAR on ASPC1 Subcutaneous Xenograft Tumors of nude mice

AsPC1-luciferase cells were digested and counted, and the cell density was adjusted to $5 \times 10^6$/ml (containing 20% matrigel). Six-week-old female NCG mice were purchased from Nanjing Jike Pharmaceutical Biotechnology Co., Ltd. and raised in a SPF animal house for about 1 week before subcutaneous injection of 100 ul tumor cell suspension into each mouse. Five days after tumor subcutaneous inoculation, small animal live imaging was performed and the mice were grouped as follows: NTD (uninfected T cell group, n=6), CD19-CART group (n=6), and GrB-CART group (n=6). CD19-CART and GrB-CART were prepared as described previously. T cells and CART cells were counted, and the density was adjusted to $1 \times 10^8$/ml. Each mouse was injected with 100 ul T cell or CART cell suspension via the tail vein. Subsequently, small animal live imaging was performed every 7 days and changes in tumor fluorescence signals were recorded. The experiment was terminated when the tumor volume reached about 2000 mm$^3$ and the tumor volume size was calculated.

The results shown in Figure 10 indicate that compared to the control group, GrB-CART significantly inhibits tumor growth.

## Example 13: GrB-CART Infiltration in Mouse Organs

AsPC1-luciferase cells were digested and counted, and the cell density was adjusted to $5 \times 10^6$/ml (containing 20% matrigel). Six-week-old female NCG mice were purchased from Nanjing Jike Pharmaceutical Biotechnology Co., Ltd. and raised in a SPF animal house for about 1 week before subcutaneous injection of 100 ul tumor cell suspension into each mouse. Five days after tumor subcutaneous inoculation, small animal live imaging was performed and the mice were grouped as follows: CD19-CART group (n=3), and GrB-CART group (n=3). CD19-CART and GrB-CART were prepared as described previously. CART cells were counted, and the density was adjusted to $1 \times 10^8$/ml. Each mouse was injected with 100 ul CART cell suspension via the tail vein. Tissues (subcutaneous tumor, heart, liver, spleen, lung, kidney, pancreas, and brain) were collected on the fifth day after tumor inoculation. Part of the tissues were stained with HE, and the other part was subjected to immunohistochemistry for CD3 staining to detect CART cell infiltration.

The experimental results are shown in Fig. 11. The GRB-CAR group has a significant enrichment of T cells in the transplanted tumor while the CD19-CAR group has not, and there is no significant difference in other tissues between the 2 T cell groups. HE staining results show that both CD19-CAR and GRB-CAR treatments do not cause significant damage to the major organs.

## Example 14: Inhibition on Pancreatic Cancer Metastasis by GRB-CART

AsPC1-luciferase cells were digested and counted, and the cell density was adjusted to $5\times10^6$/ml (containing 20% matrigel). Six-week-old female NCG mice were purchased from Nanjing Jike Pharmaceutical Biotechnology Co., Ltd. and raised in a SPF animal house for about 1 week before subcutaneous injection of 100 ul tumor cell suspension into each mouse. 12 days after tumor subcutaneous inoculation (when the tumor volume had reached a certain size), small animal live imaging was performed and the mice were grouped as follows: CD19-CART group (n=7), and GRB-CART group (n=7). CD19-CART and GrB-CART were prepared as described previously. T cells and CART cells were counted, and the density was adjusted to $2\times10^7$/ml. Each mouse was injected with 100 ul CART cell suspension via the tail vein. Subsequently, the mice received three additional re-injections of the same amount of CART cells every ten days. Small animal live imaging was performed simultaneously and changes in tumor fluorescence signals were recorded. Blood samples were collected from the mice at different time points before tumor cell transplantation and before and after CART cell injection, and stored at -80°C.

The experimental results are shown in Fig. 12. Multiple injections of low-dose GrB-CART cells do not significantly inhibit subcutaneous tumor growth but suppressed the formation of lung metastatic foci.

Genome was extracted from peripheral blood using phenol-chloroform, and quantified using NanoDrop one. To detect the expression of CART cells in peripheral blood, specific primers targeting CD19-CART and GrB-CART were designed for fluorescence quantification. To detect the presence of tumor cells in peripheral blood, specific primers targeting luciferase were used for fluorescence quantification.

The experimental results are shown in Fig. 13. Compared to the CD19-CART group, GrB-CART injection significantly reduces tumor cells and increases the CART cells in peripheral blood.

## Example 15: Inhibiting Effect of GrB-CAR on SMMC7721 Subcutaneous Xenograft Tumors of nude mice

AsPC1-luciferase cells were digested and counted, and the cell density was adjusted to $5\times10^6$/ml (containing 20% matrigel). Six-week-old female NCG mice were purchased from Nanjing Jike Pharmaceutical Biotechnology Co., Ltd. and raised in a SPF animal house for about 1 week before subcutaneous injection of 100 ul tumor cell suspension into each mouse. Five days after tumor subcutaneous inoculation, small animal live imaging was performed and the mice were grouped as follows: NTD (uninfected T cell group, n=6), CD19-CART group (n=6), and GrB-CART group (n=6). CD19-CART and LOX1-CART were prepared as described previously. T cells and CART cells were counted, and the density was adjusted to $1\times10^8$/ml. Each mouse was injected with 100 ul T cell or CART cell suspension via the tail vein. Subsequently, small animal live imaging was performed every 7 days and changes in tumor fluorescence signals were recorded. The experiment was terminated when the tumor volume reached about 2000 mm$^3$ and the tumor volume size was calculated.

The results shown in Fig. 14 indicate that compared to the CD19-CAR group, GRB-CART injection significantly inhibits the growth of SMMC7721 subcutaneous xenograft tumor.

## Claims

1. A chimeric antigen receptor (CAR) modified based on Granzyme B (GrB), wherein the CAR comprises an extracellular binding domain that specifically binds to a heat shock protein,
   wherein the extracellular binding domain comprises a GrB protein or a fragment thereof which has an amino acid sequence as shown in SEQ ID NO: 1, or an amino acid sequence as shown in positions 21-247 of SEQ ID NO: 1.

2. The CAR according to claim 1, wherein the heat shock protein is HSP70..

3. The CAR according to claim 1, wherein the heat shock protein is membrane-bound HSP70.

4. The CAR according to claim 1, wherein the amino acid sequence of the GrB protein or the fragment thereof is as shown in positions 21-247 of the sequence of SEQ ID NO: 1.

5. The CAR according to any one of claims 1-4, wherein the structure of the chimeric antigen receptor is shown in the following Formula I:

$$L\text{-}EB\text{-}H\text{-}TM\text{-}C\text{-}CD3\zeta\text{-}RP \qquad (I)$$

wherein,

each "-" is independently a linking peptide or peptide bond;
L is absent or a signal peptide sequence;
EB is an extracellular binding domain;
H is absent or a hinge region;
TM is a transmembrane domain;
C is absent or a co-stimulatory signaling molecule;
CD3ζ is a cytoplasmic signal transduction sequence derived from CD3ζ;
RP is absent or a reporter protein.

6. The CAR according to claim 5, wherein the amino acid sequence of the chimeric antigen receptor is shown in SEQ ID NO: 8.

7. A nucleic acid molecule encoding the chimeric antigen receptor according to claim 1.

8. A vector comprising the nucleic acid molecule according to claim 7.

9. A host cell comprising the vector according to claim 8, or expressing the CAR according to claim 1.

10. An engineered immune cell comprising the vector according to claim 8, or expressing the CAR according to claim 1.

11. A method for the preparation of the engineered immune cell according to claim 10, which comprises a step of transducing the nucleic acid molecule according to claim 7 or the vector according to claim 8 into the immune cell, thereby obtaining the engineered immune cell.

12. A pharmaceutical composition comprising the CAR according to claim 1, the nucleic acid molecule according to claim 7, the vector according to claim 8, the host cell according to claim 9, and/or the engineered immune cell according to claim 10, and a pharmaceutically acceptable carrier, diluent or excipient.

13. The CAR according to claim 1, the nucleic acid molecule according to claim 7, the vector according to claim 8, the host cell according to claim 9, and/or the engineered immune cell according to claim 10, for use in preventing and/or treating a disease expressing HSP70.

14. The CAR, the nucleic acid molecule, the vector, the host cell and/or the engineered immune cell for the use of claim 13, wherein the disease expressing HSP70 is selected from the group consisting of: pancreatic cancer, liver cancer, prostate cancer, acute myeloid leukemia, breast cancer, endometrial cancer, and rectal cancer.


**Patentansprüche**

1. Chimärer Antigenrezeptor (CAR), der auf der Basis von Granzyme B (GrB) modifiziert ist, wobei der CAR eine extrazelluläre Bindungsdomäne umfasst, die spezifisch an ein Hitzeschockprotein bindet,
wobei die extrazelluläre Bindungsdomäne ein GrB-Protein oder ein Fragment davon umfasst, das eine Aminosäuresequenz aufweist, wie gezeigt in SEQ ID NO: 1, oder eine Aminosäuresequenz wie gezeigt in den Positionen 21 bis 247 von SEQ ID NO: 1.

2. CAR nach Anspruch 1, wobei das Hitzeschockprotein HSP70 ist.

3. CAR nach Anspruch 1, wobei das Hitzeschockprotein membrangebundenes HSP70 ist.

4. CAR nach Anspruch 1, wobei die Aminosäuresequenz des GrB-Proteins oder des Fragments davon ist wie gezeigt in den Positionen 21 bis 247 der Sequenz von SEQ ID NO: 1.

5. CAR nach einem der Ansprüche 1 bis 4, wobei die Struktur des chimären Antigenrezeptors gezeigt ist in der folgenden Formel I:

$$L\text{-}EB\text{-}H\text{-}TM\text{-}C\text{-}CD3\zeta\text{-}RP \qquad (I)$$

wobei

jedes "-" unabhängig voneinander ein Verknüpfungspeptid oder eine Peptidbindung ist;
L fehlt oder eine Signalpeptidsequenz ist;
EB eine extrazelluläre Bindungsdomäne ist;
H abwesend oder eine Gelenkregion ist;
TM eine Transmembrandomäne ist;
C fehlt oder ein co-stimulatorisches Signalmolekül ist;
CD3ζ eine zytoplasmatische Signaltransduktionssequenz ist, die von CD3ζ abgeleitet ist;
RP abwesend oder ein Reporterprotein ist.

6. CAR nach Anspruch 5, wobei die Aminosäuresequenz des chimären Antigenrezeptors gezeigt ist in SEQ ID NO: 8.

7. Nukleinsäuremolekül, das den chimären Antigenrezeptor nach Anspruch 1 codiert.

8. Vektor, umfassend das Nukleinsäuremolekül nach Anspruch 7.

9. Wirtszelle, umfassend den Vektor nach Anspruch 8 oder exprimierend den CAR nach Anspruch 1.

10. Gentechnisch hergestellte Immunzelle, umfassend den Vektor nach Anspruch 8 oder exprimierend den CAR nach Anspruch 1.

11. Verfahren zur Vorbereitung der gentechnisch hergestellten Immunzelle nach Anspruch 10, das einen Schritt der Transduktion des Nukleinsäuremoleküls nach Anspruch 7 oder des Vektors nach Anspruch 8 in die Immunzelle umfasst, wodurch die gentechnisch hergestellte Immunzelle erhalten wird.

12. Pharmazeutische Zusammensetzung, umfassend den CAR nach Anspruch 1, das Nukleinsäuremolekül nach Anspruch 7, den Vektor nach Anspruch 8, die Wirtszelle nach Anspruch 9 und/oder die gentechnisch hergestellte Immunzelle nach Anspruch 10 und einen pharmazeutisch verträglichen Träger, Verdünnungsmittel oder Hilfsstoff.

13. CAR nach Anspruch 1, Nukleinsäuremolekül nach Anspruch 7, Vektor nach Anspruch 8, Wirtszelle nach Anspruch 9 und/oder gentechnisch hergestellte Immunzelle nach Anspruch 10 zur Verwendung bei der Vorbeugung und/oder Behandlung einer Krankheit, die HSP70 exprimiert.

14. CAR, Nukleinsäuremolekül, Vektor, Wirtszelle und/oder gentechnisch hergestellte Immunzelle zur Verwendung nach Anspruch 13, wobei die Krankheit, die HSP70 exprimiert, ausgewählt ist aus der Gruppe bestehend aus: Bauchspeicheldrüsenkrebs, Leberkrebs, Prostatakrebs, akute myeloische Leukämie, Brustkrebs, Endometriumkrebs und Rektumkarzinom.

**Revendications**

1. Récepteur antigénique chimérique (CAR) modifié sur la base du granzyme B (GrB), dans lequel le CAR comprend un domaine de liaison extracellulaire qui se lie spécifiquement à une protéine de choc thermique,
dans lequel le domaine de liaison extracellulaire comprend une protéine GrB ou un fragment de celle-ci qui présente une séquence d'acides aminés telle que présentée dans SEQ ID NO: 1, ou une séquence d'acides aminés telle que présentée aux positions 21 à 247 de SEQ ID NO: 1.

2. CAR selon la revendication 1, dans lequel la protéine de choc thermique est HSP70.

3. CAR selon la revendication 1, dans lequel la protéine de choc thermique est une HSP70 liée à la membrane.

4. CAR selon la revendication 1, dans laquelle la séquence d'acides aminés de la protéine GrB ou le fragment de celle-ci est telle que présentée aux positions 21 à 247 de la séquence de SEQ ID NO: 1.

5. CAR selon l'une quelconque des revendications 1 à 4, dans lequel la structure du récepteur antigénique chimérique est présentée dans la Formule I suivante :

$$L\text{-}EB\text{-}H\text{-}TM\text{-}C\text{-}CD3\zeta\text{-}RP \qquad (1)$$

où

chaque « - » est indépendamment un peptide de liaison ou une liaison peptidique ;
L est absent ou est une séquence de peptide signal ;
EB est un domaine de liaison extracellulaire ;
H est absent ou une région charnière ;
TM est un domaine transmembranaire ;
C est absent ou est une molécule de signalisation costimulante ;
CD3$\zeta$ est une séquence de transduction de signal cytoplasmique dérivée de CD3$\zeta$ ;
RP est absent ou une protéine rapportrice.

6. CAR selon la revendication 5, dans lequel la séquence d'acides aminés du récepteur antigénique chimérique est présentée dans SEQ ID NO: 8.

7. Molécule d'acide nucléique codant pour le récepteur antigénique chimérique selon la revendication 1.

8. Vecteur comprenant la molécule d'acide nucléique selon la revendication 7.

9. Cellule hôte comprenant le vecteur selon la revendication 8, ou exprimant le CAR selon la revendication 1.

10. Cellule immunitaire modifiée comprenant le vecteur selon la revendication 8, ou exprimant le CAR selon la revendication 1.

11. Procédé pour la préparation de la cellule immunitaire modifiée selon la revendication 10, qui comprend une étape de transduction de la molécule d'acide nucléique selon la revendication 7 ou du vecteur selon la revendication 8 dans la cellule immunitaire, obtenant ainsi la cellule immunitaire modifiée.

12. Composition pharmaceutique comprenant le CAR selon la revendication 1, la molécule d'acide nucléique selon la revendication 7, le vecteur selon la revendication 8, le cellule hôte selon la revendication 9, et/ou la cellule immunitaire modifiée selon la revendication 10 et un véhicule, diluant ou excipient pharmaceutiquement acceptable.

13. CAR selon la revendication 1, molécule d'acide nucléique selon la revendication 7, vecteur selon la revendication 8, cellule hôte selon la revendication 9 et/ou cellule immunitaire modifiée selon la revendication 10, pour une utilisation dans la prévention et/ou le traitement d'une maladie exprimant HSP70.

14. CAR, molécule d'acide nucléique, vecteur, cellule hôte et/ou cellule immunitaire modifiée pour l'utilisation selon la revendication 13, dans lesquels la maladie exprimant HSP70 est choisie dans le groupe constitué de : cancer du pancréas, cancer du foie, cancer de la prostate, leucémie myéloïde aiguë, cancer du sein, cancer de l'endomètre et cancer du rectum.

Fig. 1

Fig. 2

Fig. 3

Fig. 4

Fig. 5

Fig. 6

Fig. 7

Fig. 8

Fig. 9



Fig. 10

Fig. 11

Fig. 12

A:

B:

C:

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016120217 A1
- US 5399346 A
- US 5580859 A
- US 5589466 A
- WO 0196584 A
- WO 0129058 A
- US 6326193 B
- US 5350674 A
- US 5585362 A

**Non-patent literature cited in the description**

- **SAMBROOK et al.** Molecular Cloning: A Laboratory Manual. Cold Spring Harbor Laboratory, 2001
- **UI-TEI et al.** *FEBS Letters*, 2000, vol. 479, 79-82
- **ROSENBERG et al.** *New Eng. J. of Med.*, 1988, vol. 319, 1676
- **SAMBROOK et al.** Molecular Cloning: Laboratory Manual. Cold Spring Harbor Laboratory Press, 1989